# EUROPEAN PATENT SPECIFICATION

(11) **EP 2 207 569 B1**
(45) Date of publication and mention of the grant of the patent: **27.03.2013**
(21) Application number: 08801937.7
(22) Date of filing: 09.09.2008
(51) Int. Cl.: A61K 31/51, A61K 45/06, A61K 31/195, A61P 29/00

(54) **PHARMACEUTICAL COMPOSITIONS CONTAINING BENFOTIAMINE AND ONE OR MORE PHARMACEUTICALLY ACTIVE AGENTS FOR THE TREATMENT OF PAIN CONDITIONS OF NEUROPATHIC ORIGIN**
PHARMAZEUTISCHE ZUSAMMENSETZUNGEN MIT BENFOTIAMIN UND EINEM ODER MEHREREN WIRKSTOFFEN ZUR BEHANDLUNG NEUROPATHISCH BEDINGTER SCHMERZEN
COMPOSITIONS PHARMACEUTIQUES CONTENANT DE LA BENFOTIAMINE ET UN OU PLUSIEURS AGENTS PHARMACEUTIQUEMENT ACTIFS POUR LE TRAITEMENT D'ÉTATS DE DOULEUR D'ORIGINE NÉVROPATHIQUE

(30) Priority: 09.10.2007 EP 07019693
(43) Date of publication of application: 21.07.2010
(73) Proprietor: Merck Patent GmbH, 64271 Darmstadt (DE)
(72) Inventor: BONKE, Dieter, 64380 Rossdorf (DE); MEDINA-SANTILLÁ, Roberto, México D.F. C.P.11560 (MX); REYES-GARCIA, Gerardo, México D.F. C.P. 03710 (MX)
(86) International application number: PCT/EP2008/007364
(87) International publication number: WO 2009/046801

(56) References cited:
- WO-A-03/053456
- DATABASE WPI Week 199339 Thomson Scientific, London, GB; AN 1993-309119 XP002512439 & JP 05 221857 A (ARAKUSU KK) 31 August 1993 (1993-08-31)
- SURCHEVA S ET AL: "Benfotiamine modulates analgesic effect of COX inhibitors. Studies on nociceptive perception and inflammatory hyperalgesia" BULGARSKA AKADEMIYA NA NAUKITE. DOKLADI, AKADEMICHNO IZDATELSTVO PROF. MARIN DRINOV, SOFIA, BG, vol. 59, no. 12, 1 January 2006 (2006-01-01), pages 1307-1312, XP009109277

## Description

### Field of the invention

The present invention relates to pharmaceutical compositions containing benfotiamine and gabapentin, the process for their preparation and their use for the treatment and prevention of conditions and diseases selected from the group consisting of pain condititions of neuropathic origin.

### Background of the invention

Mixtures of two analgesic agents are often used in therapeutics in an attempt to produce greater analgesic effects while reducing side effects. In addition, the multiplicity of mechanisms involved in pain (Millan, M.J.: Prog. Neurobiol. 57: 1-164 (1999) suggests that combination therapy may improve pain management. Previous experiments in animals have shown that vitamins B₁ (thiamin), B₆ (pyridoxine) and B₁₂ (cyanocobalamin) and their combination have antinociceptive activity against chemical- and heat-induced pain (Bartoszyk, G.D. and Wild, A.: Neurosci. Lett. 101: 95-100 (1989); Franqa, D.S. et al.: Eur. J. Pharmacol. 421: 157-164 (2001); Reyes-Garcia, G. et al.: Proc. West. Pharmacol. Soc. 44: 139-140 (2001); Reyes-Garcia, G., et al.: Proc. West. Pharmacol. Soc. 45: 144-146 (2002)).

Moreover, the individual administration of vitamins B₁ and B₆ as well as riboflavin produce antinociception in acetic acid induced pain or pain induced by supramaximal electrical stimulation of afferent C fibers (Franqa, D.S. et al.: Eur. J. Pharmacol. 421:157-164 (2001); Jurna, I. et al.: Ann. N.Y. Acad. Sci. 585: 492-495 (1990)). In contrast, negative results have also been observed. Vitamin B12 was not able to produce antinociception in this model (Franqa, D.S. et al.: Eur. J. Pharmacol. 421: 157-164 (2001)). A similar result has been previously described for the failure of vitamin B₁₂, to produce antinociception in several models of pain (Eschalier, A., et al.: Psychopharmacol. 8: 228-231 (1983). In addition, neither vitamin B₆ nor B₁₂ was able to reduce neuropathic pain in rats (Misumi, J., et al.: Arch. Toxicol. 56: 204-206 (1985)). Also, benfotiamine has shown therapeutic efficacy in the treatment of painful diabetic neuropathy in human beings and in neuropathic pain models in the rat (Simeonov, S. et al., Folia Med (Plovdiv).; 39: 5-10 (1997); Sanchez-Ramirez, G. M., Eur.. J. Pharmacol., 530: 48-53 (2006)).

A synergism in the antiallodynic, antinociceptive or analgesic action was shown for combinations of gabapentin, diclofenac, dexamethasone or ketorolac with a vitamin B complex consisting of thiamine, vitamin B6 and vitamin B12 (Reyes-Garcia G., et al. Proc. West Pharmacol. Soc., 46: 91-94 (2003); Medina-Santillan, R. et al., Proc. West Pharmacol. Soc., 47:109-112, (2004); Rocha-Gonzalez, H. et al., Proc. West Pharmacol. Soc., 47: 84-87 (2004); Grandos-Soto, V. et al., Proc West Pharmacol Soc., 47:92-94 (2004); Reyes-Garcia G., et al. Proc West Pharmacol Soc.; 45:147-149 (2002); Caram-Salas N. L. et al., Proc West Pharmacol Soc., 47: 88-91 (2004); Medina-Santillan, R. et al., Proc West Pharmacol Soc., 47: 95-99 (2004)).

Gabapentin, a structural analog of γ-aminobutyric acid (GABA), is a anticonvulsant used effectively for the treatment of refractory partial seizures with and without secondarily generalized tonic-clonic seizures (Burgey, G.K., et al.: Neurol. 49: 739-745 (1997)). Several studies indicate that, besides its antiepileptic activity, gabapentin is an effective antihyperalgesic agent. Systemic or intrathecal administration of gabapentin attenuates nociceptiv behaviors in animals and pain in humans that arise following peripheral nerve injury (Xiao, W.-Y. and Bennett, G.J.: Anesth. Analg. 2: 267-270 (1996); Shimoyama, M., et al.: Neurosci Lett. 222: 65-67 (1997); Gillin, S. and Sorkin, L.S.: Anesth. Analg. 86: 111-116 (1997); Hwang, J.H. and Yaksh, T.L.: Regional Anesth. 22: 249-256 (1997); Chapman, V., et al.: Pain 75: 261-293 (1998); Pan, H.-L., et al.: J. Pharmacol. Exp. Ther. 288: 1026-1030 (1999); Chizh, B.A., et al.: Naunyn-Schmiedeberg's Arch. Pharmacol. 362: 197-200 (2000); Kaneko, M., et al.: J. Pharmacol. Exp. Ther. 292: 743-751 (2000)).

However, there is a considerable incidence of gabapentin-induced side effects at doses that produce relief of neuropathic pain in humans (Mellick, G.A. and Mellick, L.B.: J. Pain Symp. Manage. 10: 265-266 (1995); Rosner, H., et al.: Clin. J. Pain 12: 56-58 (1996); Rowbotham, M., et al.: JAMA 280: 1837-1842 (1998); Rice, A.S.C. and Maton, S.: Pain 94: 215-224 (2001). Several studies in rats have examined the incidence of side effects for this drug in relieving neuropathic pain (Chen, S.R., et al.: Anesthesiology 92: 500-506 (2000); Field, M.J., et al.: J. Pharmacol. Exp. Their. 303: 730-735 (2002)).

Thus, as there remains a continuing need in advantageous therapeutics, a preferred object of the present invention was to provide new pharmaceutical compositions for the treatment and prevention of conditions and diseases such as pain conditions of neuropathic origin, with less side-effect and better kinetics at lower doses.

The disclosure of the above-mentioned publications, does not encompass the pharmaceutical compositions of the present invention and the citation of any reference in this application is not an admission that the reference is prior art to this application.

### Summary of the invention

Surprisingly, it was found that pharmaceutical compositions containing benfotiamine and gabapentin show a strong synergistic effect in the treatment of pain conditions of neuropathic origin. This synergistic interaction is significantly higher than the synergism previously described for combinations of gabapentin, diclofenac, dexamethasone or ketorolac with a vitamin B complex consisting of thiamine, vitamin B6 and vitamin B12.

Therefore, an embodiment of the present invention are pharmaceutical compositions containing a therapeutically effective amount of benfotiamine and a therapeutically effective amount of gabapentin.

Especially preferred are pharmaceutical compositions containing a therapeutically effective amount of benfotiamine and gabapentin.

As described above, gabapentin shows considerable incidence of gabapentin-induced side effects at doses that produce relief of neuropathic pain in humans. Also pregabalin, XP13512, carbamacepin, amitryptilin, ketorolac, diclofenac, ibuprofen, flurpirtin, paracetamol and dexamethasone show undesired side effect at high doses. Gabapentin is commonly used with doses from 1800 to 3600 mg/d, i.e. 30-60 mg per kg body weight per day. Benfotiamine is commonly used with doses from 50 to 900 mg/d, i.e. 0,83-15 mg per kg body weight per day.

Surprisingly, pharmaceutical compositions according to the invention allow the administration of benfotiamine and gabapentin in unexpected lower doses. For example, pharmaceutical compositions according to the present invention containing gabapentin and benfotiamine already show a sufficient efficacy in the treatment of neuropathic pain with doses of 20-27 mg gabapentin per kg body weight per day and 10-13,5 mg benfotiamine per kg body weight per day, so that the especially the dose of gabapentin can be reduced significantly.

Therefore, the strong synergistic effect enables the physician to use lower doses of the active agents with the advantage of avoiding the known side effects of higher doses of gabapentin.

Additionally, due to its side effect, gabapentin normally is introduced within about three weeks until an effective high dose can be administered to the patient without severe side effects. Therefore, as the necessary maximal dose of the analgesic acting substance gabapentin administered with the pharmaceutical compositions of the present invention can be chosen much lower. Furthermore, the pharmaceutical compositions of the present invention allow a faster introduction of the pharmaceutically active agents and a faster therapeutic activity of the medicament.

The synergistic effect of a co-administration of benfotiamine and gabapentin might be effected by a facilated membrane transport of the analgesic acting substance gabapentin, induced by benfotiamine. Benfotiamine which, due to its lipophilic character, itself shows a much higher bioavailability in comparison to thiamine, seems to facilate the membrane transport of the accompanying agent, leading to an increased bioavailability of the analgesic acting substance gabapentin, with the advantage of reducing the required dose and a faster onset of the pharmaceutical acitivity with lower doses. That might be a similar effect as the facilated transport of drugs by lipohilic nanoparticles and might explain that the synergistic effect of a combination of benfotiamine and i.e. gabapentin is more pronounced than the synergistic effect of a combination of gabapentin, demamethasone or ketorolac with a vitamin B complex comprising thiamine, vitamin B6 and vitamin B12. This higher degree of synergism could not have been expected from the former studies.

The results reported here indicate that the combination of benfotiamine and gabapentin is able to modulate tactile allodynia induced by L5 and L6 spinal nerve ligation in a synergistivc manner. Besides, producing antiallodynia by itself, when orally co-administered with gabapentin, benfotiamine produced a functional synergism interaction in spinal nerveligated rats. To our knowledge, this is the first report demonstrating a synergistic interaction between benfotiamine and gabapentin. Surprisingly, this synergism is significantly higher than the synergistic interaction reported for the combination of gabapentin and the vitamin B complex Reyes-Garcia G., et al. Proc. West Pharmacol. Soc., 46: 91-94 (2003).

The mechanism of this antiallodynic interaction remains to be elucidated, but could be due either to a pharmacokinetic interaction or to the different mechanisms and sites of action of the individual drugs.

Therefore a further embodiment of the present invention are pharmaceutical compositions according to the present invention, characterized in that they are administered to a patient, in the need thereof with a therapeutically effective daily dose of 1 to 15 mg benfotiamine per kg body weight and a therapeutically effective daily dose of 10 to 60 mg gabapentin per kg body weight. Preferred are doses of 5 to 14 mg benfotiamine per kg body weight and 10 to 28 mg gabapentin per kg body weight, especially preferred are doses of 10 to 13,5 mg benfotiamine per kg body weight and 20 to 27 mg gabapentin per kg body weight,

The term "composition", as in pharmaceutical composition, is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

Therefore, a further embodiment of the present invention are pharmaceutical compositions according to the invention, characterized in that they contain one or more additional compounds, selected from the group consisting of physiologically acceptable excipients, auxiliaries, adjuvants, diluents, carriers and pharmaceutically active agents other than benfotiamine and gabapentin.

Within the scope of the present invention are all known physiologically acceptable salts, derivatives, prodrugs, solvates and stereoisomers of the present active pharmaceutical ingredients, including mixtures thereof in all ratios. By the term "solvate" is meant a hydrate, an alcoholate, or other solvate of crystallization.

A further embodiment of the present invention are sets (kits) consisting of separate packets of a therapeutically effective amount of benfotiamine and a therapeutically effective amount of gabapentin.

An additional embodiment of the present invention are sets (kits) according to the invention, characterized in that they consist of one or more further separate packets of pharmaceutically active agents other than benfotiamine and gabapentin.

As used herein, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. Said therapeutic effective amount of one or more of the compounds according to the invention is known to the skilled artisan or can be easily determined by standard methods known in the art.

Another aspect of the present invention is a process for the manufacture of pharmaceutical compositions according to the invention, characterized in that a therapeutically effective amount of benfotiamine, a therapeutically effective amount of gabapentin and one or more compounds selected from the group consisting of solid, liquid or semiliquid excipients, auxiliaries, adjuvants, diluents, carriers and pharmaceutically active agents other than benfotiamine and gabapentin are converted in a suitable dosage form. The present invention includes the use of pharmaceutical compositions of the present invention for the treatment, control, amelioration, prevention, delaying the onset of or reducing the risk of developing the diseases and conditions that are described herein, in a mammalian patient in need of such treatment, particularly a human, by the administration of benfotiamine and gabapentin, in an amount that is effective to treat said condition.

The terms "administration of" and "administering a" compound should be understood to mean providing a compound of the invention or a prodrug of a compound of the invention to the individualist need.

Therefore, a further preferred embodiment of the present invention is the use of the pharmaceutical compositions of the present invention for the preparation of a medicament.

A further preferred embodiment of the present invention is the use of a pharmaceutical composition of the present invention for the preparation of caused, mediated and/or propagated by lesions, dysfunction, compression and or transitory perturbations of the neuronal system.

A further preferred embodiment of the present invention is the use of a pharmaceutical composition according to the present invention for the preparation of a medicament for the treatment and/or prevention of one ore more disease or condition selected from the group consisting of pain conditions of neuropathic origin.

A further preferred embodiment of the present invention is the use of a pharmaceutical composition according to the present invention for the preparation of a medicament for the treatment and/or prevention of one ore more disease or condition selected from the group consisting of pain conditions of neuropathic origin selected from the group consisting of neuropathic pain due to lesions, dysfunctions, compressions or transitory perturbations of the neuronal system, due to diabetic neuropathy or back pain of neuropathic origin.

The pharmaceutical compositions of the present invention may be used in combination with one or more other drugs in the treatment, prevention, suppression or amelioration of diseases or conditions for which the pharmaceutical compositions of the present invention or the other drugs have utility. Often, the combination of the drugs is safer or more effective than either drug alone, or the combination is safer or more effective than would it be expected based on the additive properties of the individual drugs. Such other drug(s) may be administered by a route and in an amount commonly used contemporaneously or sequentially with a pharmaceutical composition of the present invention. When a pharmaceutical composition of the present invention is used contemporaneously with one or more other drugs, a combination product containing such other drug(s) and the pharmaceutical composition of the present invention is preferred. However, combination therapy also includes present invention is preferred. However, combination therapy also includes therapies in which the pharmaceutical composition of the present invention and one or more other drugs are administered on different overlapping schedules. It is contemplated that when used in combination with other active ingredients, the pharmaceutical composition of the present invention or the other active ingredient or both may be used effectively in lower doses than when each is used alone. Accordingly, the pharmaceutical compositions of the present invention include those that contain one or more other active ingredients, in addition to benfotiamine and gabapentin. Examples of other active ingredients may be administered in combination with a pharmaceutical composition of the present invention, and either administered separately or in the same pharmaceutical composition.

The pharmaceutical compositions of the present invention may be administered by any means that achieve their intended purpose. For example, administration may be by oral, parenteral, topical, enteral, intravenous, intramuscular, inhalant, nasal, intraarticular, intraspinal, transtracheal, transocular, subcutaneous, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired. Parenteral administration is preferred. Oral administration is especially preferred.

Suitable dosage forms include capsules, tablets, pellets, dragees, semi-solids, powders, granules, suppositories, ointments, creams, lotions, inhalants, injections, cataplasms, gels, tapes, eye drops, solution, syrups, aerosols, suspension, emulsion, which can be produced according to methods known in the art, for example as described below:
mixing of active ingredient/s and auxiliaries, compression of said mixture into tablets (direct compression), optionally granulation of part of mixture before compression.
capsules:
   mixing of active ingredient/s and auxiliaries to obtain a flowable powder, optionally granulating powder, filling powders/granulate into opened capsules, capping of capsules.
semi-solids (ointments, gels, creams):
   dissolving/dispersing active ingredient/s in an aqueous or fatty carrier; subsequent mixing of aqueous/fatty phase with complementary fatty/aqueous phase, homogenization (creams only).
suppositories (rectal and vaginal):
   dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms.
aerosols:
   dispersing/dissolving active agent/s in a propellant, bottling said mixture into an atomizer.

In general, non-chemical routes for the production of pharmaceutical compositions and/or pharmaceutical preparations comprise processing steps on suitable mechanical means known in the art that transfer one or more compounds according to the invention into a dosage form suitable for administration to a patient in need of such a treatment. Usually, the transfer of one or more compounds according to the invention into such a dosage form comprises the addition of one or more compounds, selected from the group consisting of carriers, excipients, auxiliaries and pharmaceutical group consisting of carriers, excipients, auxiliaries and pharmaceutical active ingredients other than the compounds according to the invention. Suitable processing steps include combining, milling, mixing, granulating, dissolving, dispersing, homogenizing, casting and/or compressing the respective active and non-active ingredients. Mechanical means for performing said processing steps are known in the art, for example from Ullmann's Encyclopedia of Industrial Chemistry, 5th Edition. In this respect, active ingredients are preferably at least one compound according to this invention and one or more additional compounds other than the compounds according to the invention, which show valuable pharmaceutical properties, preferably those pharmaceutical active agents other than the compounds according to the invention, which are disclosed herein.

Particularly suitable for oral use are tablets, pills, coated tablets, capsules, powders, granules, syrups, juices or drops, suitable for rectal use are suppositories, suitable for parenteral use are solutions, preferably oil-based or aqueous solutions, furthermore suspensions, emulsions or implants, and suitable for topical use are ointments, creams or powders. The novel compounds may also be lyophilised and the resultant lyophilisates used, for example, for the preparation of injection preparations. The preparations indicated may be sterilised and/or comprise assistants, such as lubricants, preservatives, stabilisers and/or wetting agents, emulsifiers, salts for modifying the osmotic pressure, buffer substances, dyes, flavours and/or a plurality of further active ingredients, for example one or more vitamins.

Suitable excipients are organic or inorganic substances, which are suitable for enteral (for example oral), parenteral or topical administration and do not react with the compounds of the present invention, for example water, vegetable oils, benzyl alcohols, alkylene glycols, polyethylene glycols, glycerol triacetate, gelatine, carbohydrates, such as lactose, sucrose, mannitol, sorbitol or starch (maize starch, wheat starch, rice starch, potato tricalcium phosphate or calcium hydrogen phosphate, magnesium stearate, talc, gelatine, tragacanth, methyl cellulose, hydroxypropylmethylcellulose, sodium carboxymethylcellulose, polyvinyl pyrrolidone and/or Vaseline.

If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries include, without limitation, flow-regulating agents and lubricants, for example, silica, talc, stearic acid or salts thereof, such as magnesium stearate or calcium stearate, and/or polyethylene glycol. Dragee cores are provided with suitable coatings, which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices or to provide a dosage form affording the advantage of prolonged action, the tablet, dragee or pill can comprise an inner dosage and an outer dosage component me latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol, solutions of suitable cellulose preparations such as acetyl-cellulose phthalate, cellulose acetate or hydroxypropymethyl-cellulose phthalate, are used. Dye stuffs or pigments may be added to the tablets or dragee coatings, for example, for identification or in order to characterize combinations of active compound doses.

Suitable carrier substances are organic or inorganic substances which are suitable for enteral (e.g. oral) or parenteral administration or topical application and do not react with the compounds of the present invention, for example water, vegetable oils, benzyl alcohols, polyethylene glycols, gelatin, carbohydrates such as lactose or starch, magnesium stearate, talc and petroleum jelly. In particular, tablets, coated tablets, capsules, syrups, suspensions, drops or suppositories are used for enteral administration, solutions, preferably oily or aqueous solutions, furthermore suspensions, emulsions or implants, are used for parenteral administration, and ointments, creams or powders are used for topical application. The novel compounds can also be lyophilized and the lyophilizates obtained can be used, for example, for the production of injection preparations.

The preparations indicated can be sterilized and/or can contain excipients such as lubricants, preservatives, stabilizers and/or wetting agents, emulsifiers, salts for affecting the osmotic pressure, buffer substances, colorants, flavourings and/or aromatizers. They can, if desired, also contain one or more further active compounds, e.g. one or more vitamins.

Other pharmaceutical preparations, which can be used orally include push-fit capsules made of gelatine, as well as soft, sealed capsules made of gelatine and a plasticizer such as glycerol or sorbitol. The push-fit capsules can contain the active compounds in the form of granules, which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatine.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400).

Aqueous injection suspensions may contain substances, which increase the viscosity of the suspension, including, for example, sodium carboxymethyl cellulose, sorbitol, and/or dextran, optionally, the suspension may also contain stabilizers.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

Possible pharmaceutical preparations, which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatine rectal capsules, which consist of a combination of the active compounds with a base. Possible base materials include, for example, liquid triglycerides, polyethylene glycols, or paraffin hydrocarbons.

For use in medicine, the compounds of the present invention will be in the form of pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic bases, e.g. quaternary ammonium salts.

The present invention includes within its scope prodrugs of the compounds of the present invention above. In general, such prodrugs will be functional derivatives of the compounds of the present invention, which are readily convertible in vivo into the required compound of the present invention. Conventional procedures for the selection and preparation of suitable prodrug derivatives are described, for example, in Design of Prodrugs, ed. H. Bundgaard, Elsevier, 1985.

The pharmaceutical preparations can be employed as medicaments in human and veterinary medicine. As used herein, the term "effective amount" means that amount of a drug or pharmaceutical agent that will elicit the biological or medical response of a tissue, system, animal or human that is being sought, for instance, by a researcher or clinician.

The substances according to the invention are generally administered analogously to commercial preparations. Due to the described synergistic effect the daily doses can be significantly reduced in order to reduce the side effects of the individual pharmaceutically active agents.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

The host, or patient, may be from any mammalian species, e.g., primate sp., particularly human; rodents, including mice, rats and hamsters; rabbits; equines, bovines, canines, felines; etc. Animal models are of interest for experimental investigations, providing a model for treatment of human disease.

The specific dose for the individual patient depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician, which advises or attends the therapeutic treatment.

In the case of many disorders, the susceptibility of a particular cell to treatment with the subject compounds may be determined by in vitro testing. Typically a culture of the cell is combined with a subject compound at varying concentrations for a period of time sufficient to allow the active agents to show a relevant reaction, usually between about one hour and one week. For in vitro testing, cultured cells from a biopsy sample may be used.

Even without further details, it is assumed that a person skilled in the art will be able to utilise the above description in the broadest scope. The preferred embodiments should therefore merely be regarded as descriptive disclosure, which is absolutely not limiting in any way.

Above and below, all temperatures are indicated in °C. In the following examples, "conventional work-up" means that, if necessary, the solvent is removed, water is added if necessary, the pH is adjusted, if necessary, to between 2 and 10, depending on the constitution of the end product, the mixture is extracted with ethyl acetate or dichloromethane, the phases are separated, the organic phase is washed with saturated NaHCO₃ solution, if desired with water and saturated NaCl solution, is dried over sodium sulfate, filtered and evaporated, and the product is purified by chromatography on silica gel, by preparative HPLC and/or by crystallization. The purified compounds are, if desired, freeze-dried. Mass spectrometry (MS): ESI (electrospray ionisation) (M+H)⁺

List of Abbreviations and Acronyms:
AcOH acetic acid, anh anhydrous, atm atmosphere(s), BOC tert-butoxycarbonyl CDI 1,1'-carbonyl diimidazole, conc concentrated, d day(s), dec decomposition, DMAC NN-dimethylacetamide, DMPU 1,3-dimethyl-3,4,5,6-tetrahydro-2(IH)-pyrimidinone, DMF NN-dimethylformamide, DMSO dimethylsulfoxide, DPPA diphenylphosphoryl azide, EDCI 1-(3-dimethylaminopropyl)-3-ethylcarbodiimide, EtOAc ethyl acetate, EtOH ethanol (100%), Et₂O diethyl ether, Et₃N triethylamine, h hour(s), MeOH methanol, pet. ether petroleum ether (boiling range 30-60°C), temp. temperature, THF tetrahydrofuran, TFA trifluoroAcOH, Tf trifluoromethanesulfonyl.

### Example 1: Allodynic activity of a combination of gabapentin and benfotiamine

The purpose of this study was to assess the possible synergistic interaction between gabapentin and benfotiamine in a neuropathic pain model in the rat (Kim, S.H. and Chung, J.M.: Pain 50: 355-363 (1992)). In addition, the possible synergistic interaction between gabapentin and benfotiamine was also assessed in rats with neuropathic pain. Neuropathic pain was induced by ligation of the left L5 and L6 spinal nerves of female Wistar rats. Tactile allodynia was determined by measuring paw withdrawal in response to probing with a series of calibrated von Frey filaments.

### Animals:

Female Wistar rats aged 6-7 weeks (weight range, 120-140 g) from our own breeding facilities were used in this study. Animals had free access to food and drinking water before experiments. Efforts were made to minimize animal suffering and to reduce the number of animals used. All experiments followed the Guidelines on Ethical Standards for Investigation of Experimental Pain in Animals (Zimmermann. M.: Pain 16: 109-110 (1983)). Additionally, the study was approved by the Institutional Animal Care and Use Committee (Centro de Investigacion y de Estudios Avanzados del Instituto Politecnico Nacional, Mexico, D.F., Mexico).

**Evaluation of antiallodynic activity:** Rats were prepared according to the method of Kim and Chung (Kim, S.H. and Chung, J.M.: Pain 50: 355-363 (1992)). Animals were anesthetized with a mixture of ketamine/xylazine (45-12 mg/kg, i.p.). After surgical preparation and exposure of the dorsal vertebral column, the L5 and L6 spinal nerves were exposed and tightly ligated with 6-0 silk suture distal to the dorsal root ganglion. For sham operated rats, the nerves were exposed but not ligated. The incisions were closed, and the animals were allowed to recover for 15 days. Rats exhibiting motor deficiency (such as paw-dragging) were discarded from testing. Tactile allodynia was determined by measuring paw withdrawal in response to probing with a series of calibrated fine filaments (von Frey filaments). The strength of the von Frey stimuli range from 0.4 to 15 g. Withdrawal thresholds were determined by increasing and decreasing stimulus strength eliciting paw withdrawal (Chaplan, S.R., et al.: J. Neurosci Meth. 53: 55-63 (1994)). All nerve-ligated rats were verified to be allodynic (responding to a stimulus of less than 4 g). Rats not demonstrating allodynia were not further studied.

**Drugs:** Gabapentin was purchased from Research Biochemicals International (Natick, MA, USA). Benfotiamine was purchased from Sigma. Gabapentin and benfotiamine were dissolved in saline and given at a volume ratio of 4 ml/kg. Study design: Rats received the oral administration of saline or increasing doses of gabapentin (30-300 mg/kg), benfotiamine (75-300 mg/kg) or the combination of gabapentin and benfotiamine 60 min before evaluation of threshold in spinal nerve injured rats. Observer was unaware of the treatment in each animal. Rats in all groups were tested for possible side effects such as reduction of righting, stepping, corneal and pinna reflexes before and after drug treatment.

**Data analysis and statistics:** Curves were constructed plotting the threshold for paw withdrawal as a function of time. Threshold for paw withdrawal was also expressed as the % of antiallodynia. % antiallodynia was calculated with the following equation: % antiallodynia = ((area under the withdrawal theshold against time curve (AUC) of the treatment - AUC saline)/(AUC sham - AUC saline) X 100. The dose-response curves were fitted using least-squares linear regression and ED₃₀ and its standard error (SEM) were calculated according to the method described by Tallarida (Tallarida, R.J.: Drug Synergism and Dose-Efect Data Analysis. 1st Ed., New York, Chapman and Hall/CRC, pp 1-72 (2000)).

For evaluation of the interaction between gabapentin and benfotiamine, isobolograms were constructed using ED₃₀ values obtained when the drugs were administered alone or combined. We used ED₃₀ instead of ED₅₀ values because benfotiamine was not able to reach more than 30% of antiallodynia in the model. The construction of the dose-response curves and the determination of doses producing 30% of antiallodynia were computed. To perform the isobolographic analysis, antiallodynic drugs were administered in combination as fixed ratios of equieffective ED₃₀ for each drug (gabapentin/benfotiamine=0.5:0.5). The ED₃₀ values (± SEM) for gabapentin and benfotiamine alone after the different administrations were plotted on the "x" and "y" axes, respectively, and the theoretical additive point was calculated according to Tallarida (Tallarida, R.J.: Drug Synergism and Dose-Efect Data Analysis. 1st Ed., New York, Chapman and Hall/CRC, pp 1-72 (2000)).

Dose-response data were analyzed by one-way analysis of variance (ANOVA) with Tukey's test for post hoc comparison. Statistical significance between the theoretical additive point and the experimentally derived ED₃₀ value was evaluated using Student's *t*-test. Differences are considered to be significant when the critical value reaches a level of P < 0.05. An experimental ED₃₀ significantly less than the theoretical additive ED₃₀ was considered to indicate a synergistic interaction between oral gabapentin and benfotiamine.

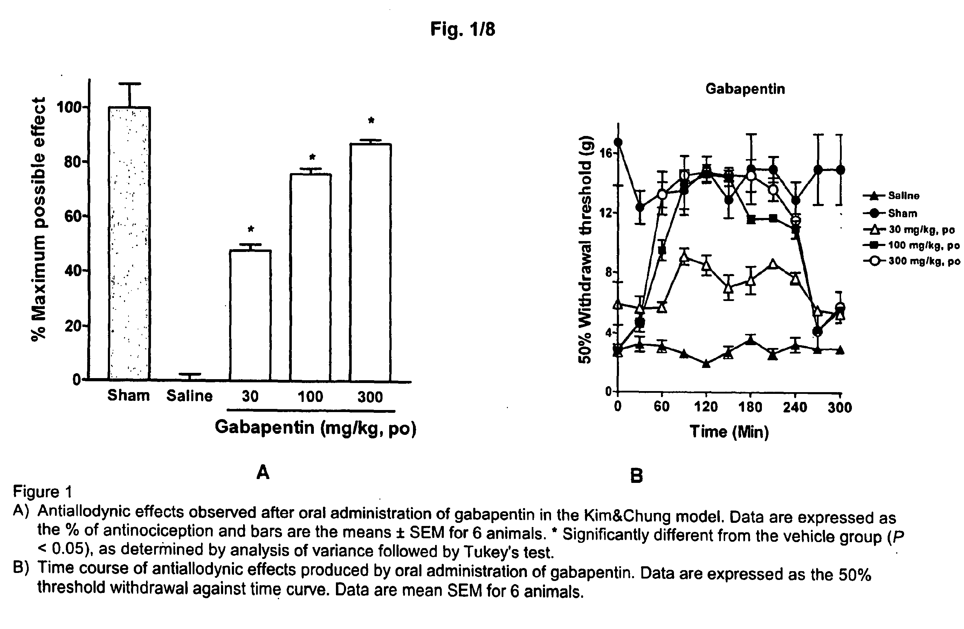

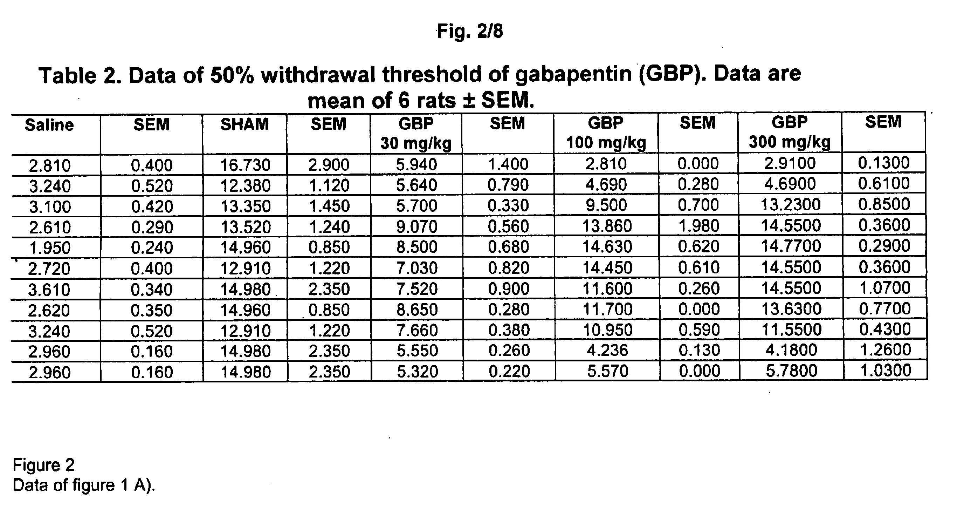

**Results:** Ligation of L5 and L6 spinal nerves produced a clear-cut allodynia in rats submitted to the surgery compared to the sham operated rats. Ligation of spinal nerves did not modify weight gain in these rats compared to the sham operated rats (data not shown). Oral administration of gabapentin, but not vehicle (saline), reduced in a dose-dependent manner, tactile allodynia (P < 0.05) induced by ligation of L5 and L6 nerves. Gabapentin produced a maximal antiallodynic effect (100%) at 300 mg/kg (ED₃₀ 16,6 ± 2,6 mg/kg) (Fig. 1A and 2). Fig. 1B shows the time course of antiallodynic effects produced by oral administration of gabapentin. No reduction in the assessed reflexes was observed in either group, control or treated (data not shown).

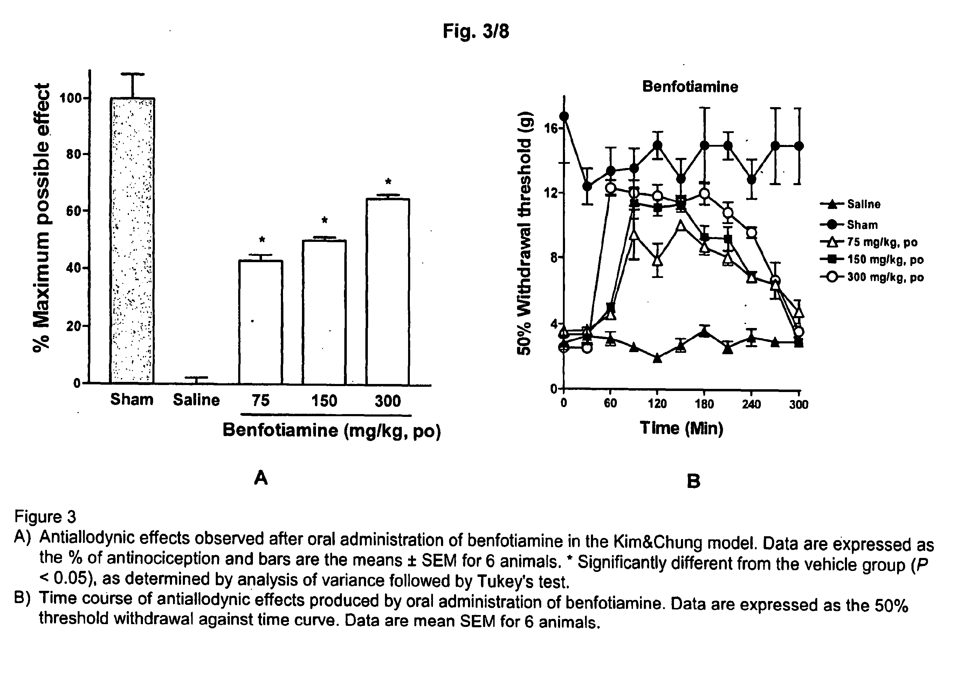

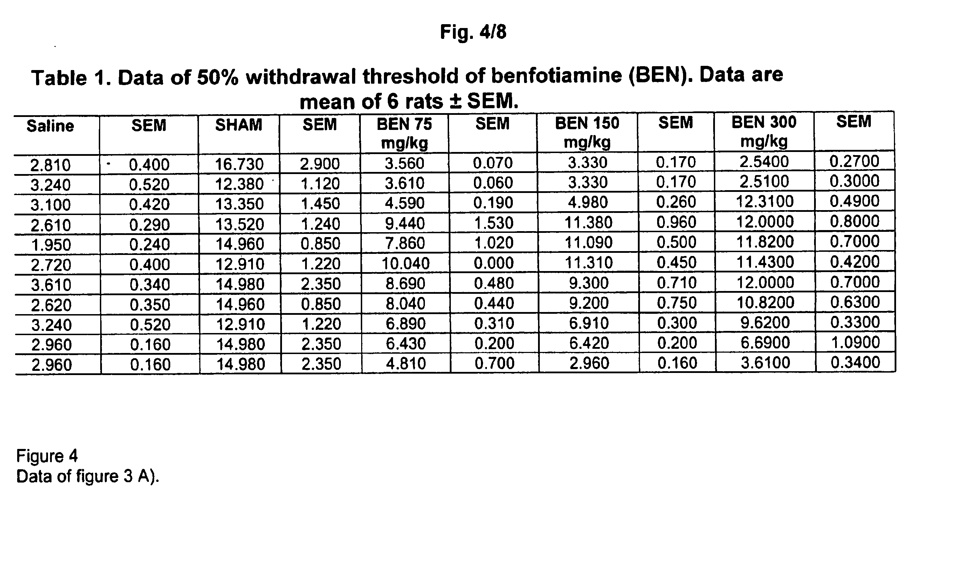

Oral administration of benfotiamine induced a modest dose-dependent reduction of tactile allodynia (P < 0.05) in spinal nerve ligated rats. Benfotiamine produced a maximal antiallodynic effect at 300 mg/kg (ED₃₀ 47,4 ± 10 mg/kg) (Fig. 3A and 4). Fig. 3B shows the time course of antiallodynic effects produced by oral administration of benfotiamine. At the selected doses, there was no evidence of modification of assessed reflexes.

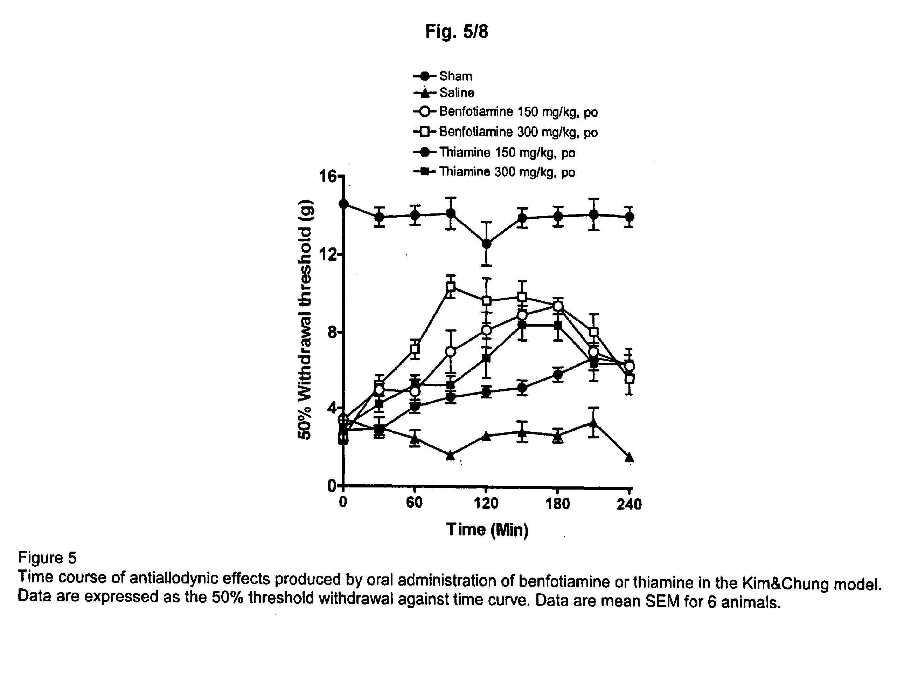

Oral administration of benfotiamine shows a higher antiallodynic effect than thiamine (Fig. 5).

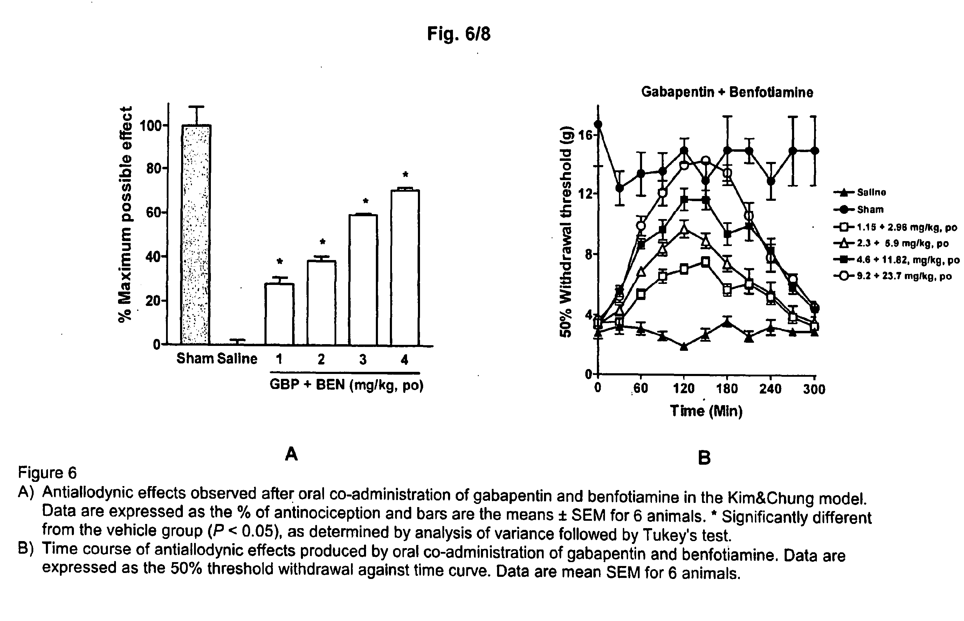

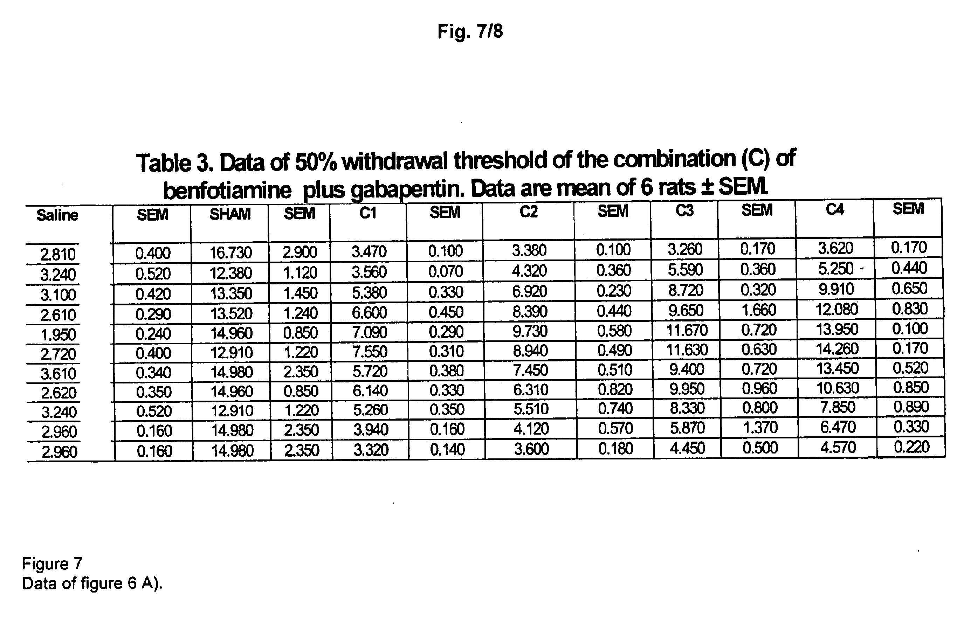

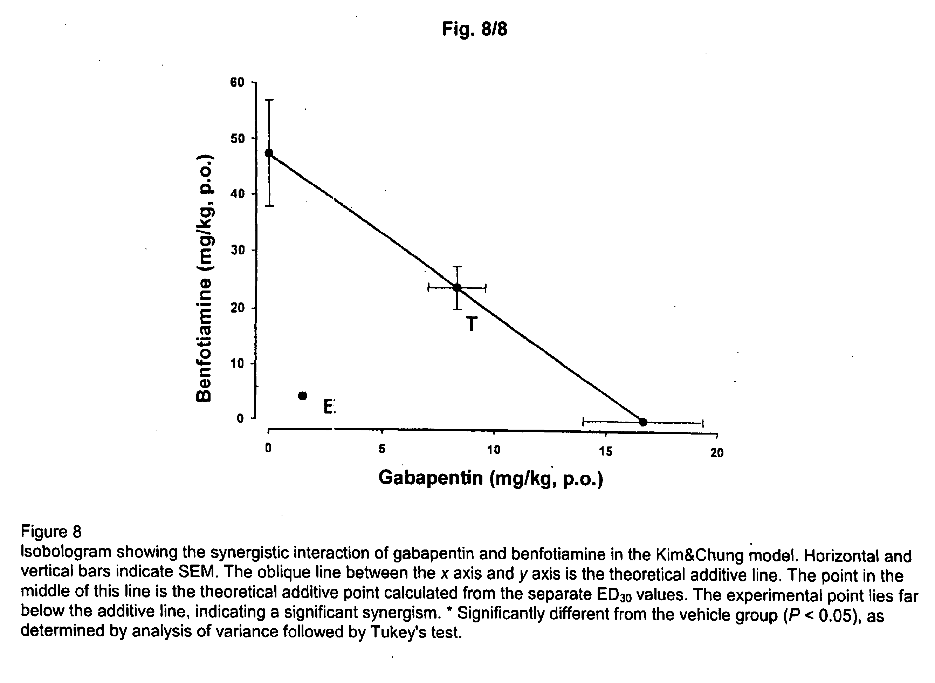

Oral co-administration of gabapentin and benfotiamine produced a dose-dependent and significant (*P* < 0.05) reduction of tactile allodynia in the spinal nerve ligated rats (Fig. 6A, 6B and 7). As shown in figure 8, the experimentally derived ED₃₀ (gabapentin 1,37 mg per kg / benfotiamine 5,4 mg per kg) of the gabapentin-benfotiamine combination is below the theoretically additive dose line (gabapentin 9,6 ± 1,3 mg / benfotiamine 22± 3,6 per kg).

### Example 2: Injection vials

A solution of 100 g of a pharmaceutical composition of the present invention and 5 g of disodium hydrogenphosphate is adjusted to pH 6.5 in 3 l of double-distilled water using 2N hydrochloric acid, sterile-filtered, dispensed into injection vials, lyophilized under sterile conditions and aseptically sealed. Each injection vial contains 5 mg of active compound.

### Example 3: Suppositories

A mixture of 20 g of a pharmaceutical composition of the present invention is fused with 100 g of soya lecithin and 1400 g of cocoa butter, poured into moulds and allowed to cool. Each suppository contains 20 mg of active compound.

### Example 4: Solution

A solution of 1 g of a pharmaceutical composition of the present invention, 9.38 g of NaH₂PO₄ · 2 H₂O, 28.48 g of Na₂HPO₄ ·12 H₂O and 0.1 g of benzalkonium chloride in 940 ml of double-distilled water is prepared. It is adjusted to pH 6.8, made up to 1 I and sterilized by irradiation. This solution can be used in the form of eye drops.

### Example 5: Ointment

500 mg of a pharmaceutical composition of the present invention is mixed with 99.5 g of petroleum jelly under aseptic conditions.

### Example 6: Tablets

A mixture of 1 kg of a pharmaceutical composition of the present invention, 4 kg of lactose, 1.2 kg of potato starch, 0.2 kg of talc and 0.1 kg of magnesium stearate is compressed to give tablets in a customary manner such that each tablet contains 10 mg of active compound.

### Example 7: Coated tablets

Analogously to the previous example, tablets are pressed and are then coated in a customary manner using a coating of sucrose, potato starch, talc, tragacanth and colourant.

### Example 8: Capsules

2 kg of a pharmaceutical composition of the present invention are dispensed into hard gelatin capsules in a customary manner such that each capsule contains 20 mg of the active compound.

## Claims

1. Pharmaceutical composition, **characterized in that** it contains a therapeutically effective amount of gabapentin and a therapeutically effective amount of benfotiamine.

2. Pharmaceutical composition according to claim 1, **characterized in that** it is suitable to be administered to a patient, in the need thereof with a therapeutically effective daily dose of 5-14 mg benfotiamine per kg body weight and a therapeutically effective daily dose of 10-28 mg gabapentin per kg body weight.

3. Pharmaceutical composition according to claim 1 or 2, **characterized in that** it contains one or more additional compounds, selected from the group consisting of physiologically acceptable excipients, auxiliaries, adjuvants, diluents, carriers and pharmaceutically active agents other than benfotiamine and gabapentin.

4. Set (kit) consisting of separate packets of
a) a therapeutically effective amount of benfotiamine and
b) a therapeutically effective amount of gabapentin.

5. Set (kit) according to claim 4, **characterized in that** it consists of one or more further separate packets of pharmaceutically active agents other than benfotiamine and gabapentin.

6. Process for the manufacture of a pharmaceutical composition according to one of the claims 1 to 3, **characterized in that** a therapeutically effective amount of benfotiamine, a therapeutically effective amount of gabapentin and one or more compounds selected from the group consisting of solid, liquid or semiliquid excipients, auxiliaries, adjuvants, diluents, carriers and pharmaceutically active agents other than benfotiamine and gabapentin are converted in a suitable dosage form.

7. A pharmaceutical composition according to one of the claims 1 to for use in the preparation of a medicament.

8. Use of a pharmaceutical composition according to one of the claims 1 to 3 for the preparation of a medicament for the treatment and/or prevention of one ore more disease or condition selected from the group consisting of of pain conditions of neuropathic origin selected from the group consisting of neuropathic pain due to lesions, dysfunctions, compressions or transitory perturbations of the neuronal system, due to diabetic neuropathy or back pain of neuropathic origin.

## Patentansprüche

1. Pharmazeutische Zusammensetzung, **dadurch gekennzeichnet, dass** sie eine therapeutisch wirksame Menge Gabapentin und eine therapeutisch wirksame Menge Benfotiamin enthält.

2. Pharmazeutische Zusammensetzung nach Anspruch 1, **dadurch gekennzeichnet, dass** sie für die Verabreichung an einen Patienten, der dessen bedarf, geeignet ist, mit einer therapeutisch wirksamen Tagesdosis von 5-14 mg Benfotiamin pro kg Körpergewicht und einer therapeutisch wirksamen Tagesdosis von 10-28 mg Gabapentin pro kg Körpergewicht.

3. Pharmazeutische Zusammensetzung nach Anspruch 1 oder 2, **dadurch gekennzeichnet, dass** sie eine oder mehrere zusätzliche Verbindungen enthält, die aus der Gruppe ausgewählt werden, die aus physiologisch unbedenklichen Excipienten, Hilfsstoffen, Adjuvantien, Verdünnungsmitteln, Trägern und anderen pharmazeutischen Wirkstoffen als Benfotiamin und Gabapentin besteht.

4. Satz (Kit) aus verschiedenen Packungen mit
a) einer therapeutisch wirksamen Menge Benfotiamin und
b) einer therapeutisch wirksamen Menge Gabapentin.

5. Satz (Kit) nach Anspruch 4, **dadurch gekennzeichnet, dass** er aus einer oder mehreren weiteren getrennten Packungen mit anderen pharmazeutischen Wirkstoffen als Benfotiamin und Gabapentin besteht.

6. Verfahren zur Herstellung einer pharmazeutischen Zusammensetzung nach einem der Ansprüche 1 bis 3, **dadurch gekennzeichnet, dass** eine therapeutisch wirksame Menge Benfotiamin, eine therapeutisch wirksame Menge Gabapentin und eine oder mehrere Verbindungen, die aus der Gruppe ausgewählt werden, die aus festen, flüssigen oder halbflüssigen Excipienten, Hilfsstoffen, Adjuvantien, Verdünnungsmitteln, Trägern und anderen pharmazeutischen Wirkstoffen als Benfotiamin und Gabapentin besteht, in eine geeignete Dosierungsform umgewandelt werden.

7. Pharmazeutische Zusammensetzung nach einem der Ansprüche 1 bis 3 für die Verwendung zur Herstellung eines Arzneimittels.

8. Verwendung einer pharmazeutischen Zusammensetzung nach einem der Ansprüche 1 bis 3 zur Herstellung eines Arzneimittels für die Behandlung und/oder Vorbeugung von einer oder mehreren Erkrankungen oder einem oder mehreren Zuständen, die aus der Gruppe ausgewählt werden, bestehend aus Schmerzzuständen neuropathischen Ursprungs, ausgewählt aus der Gruppe, die aus neuropathischem Schmerz aufgrund von Läsionen, Dysfunktionen, Kompressionen oder vorübergehenden Störungen des neuronalen Systems, aufgrund von diabetischer Neuropathie oder Rückenschmerz neuropathischen Ursprungs besteht.

## Revendications

1. Composition pharmaceutique, **caractérisée en ce qu'**elle contient une quantité thérapeutiquement efficace de gabapentine et une quantité thérapeutiquement efficace de benfotiamine.

2. Composition pharmaceutique selon la revendication 1, **caractérisée en ce qu'**elle est convenable pour être administrée à un patient en ayant besoin, selon une dose journalière thérapeutiquement efficace de 5-14 mg de benfotiamine par kg de poids corporel et une dose journalière thérapeutiquement efficace de 10-28 mg de gabapentine par kg de poids corporel.

3. Composition pharmaceutique selon la revendication 1 ou 2, **caractérisée en ce qu'**elle contient un ou plusieurs composés supplémentaires, choisis dans le groupe constitué par les excipients, les auxiliaires, les adjuvants, les diluants et les véhicules physiologiquement acceptables et des agents pharmaceutiquement actifs autres que la benfotiamine et la gabapentine.

4. Ensemble (kit) constitué de paquets séparés
a) d'une quantité thérapeutiquement efficace de benfotiamine, et
b) d'une quantité thérapeutiquement efficace de gabapentine.

5. Ensemble (kit) selon la revendication 4, **caractérisé en ce qu'**il est constitué d'un ou plusieurs autres paquets séparés d'agents pharmaceutiquement actifs autres que la benfotiamine et la gabapentine.

6. Procédé d'élaboration d'une composition pharmaceutique selon l'une quelconque des revendications 1 à 3, **caractérisé en ce qu'**une quantité thérapeutiquement efficace de benfotiamine, une quantité thérapeutiquement efficace de gabapentine, et un ou plusieurs composés choisis dans le groupe constitué par les excipients, les auxiliaires, les adjuvants, les diluants, les véhicules, solides, liquides ou semi-liquides, et des agents pharmaceutiquement actifs autres que la benfotiamine et la gabapentine, sont convertis en une forme de dosage convenable.

7. Composition pharmaceutique selon l'une quelconque des revendications 1 à 3, pour une utilisation dans la préparation d'un médicament.

8. Utilisation d'une composition pharmaceutique selon l'une quelconque des revendications 1 à 3, pour la préparation d'un médicament destiné au traitement et/ou à la prévention d'une ou plusieurs maladies ou conditions choisies dans le groupe constitué par les conditions douloureuses d'origine neuropathique choisies dans le groupe constitué par la douleur neuropathique due à des lésions, des dysfonctionnements, des compressions ou des perturbations transitoires du système neuronal, due à une neuropathie diabétique ou une douleur du dos d'origine neuropathique.
